# FASCICULE DE BREVET EUROPEEN

(11) **EP 1 549 298 B1**
(45) Date de publication et mention de la délivrance du brevet: **25.06.2008**
(21) Numéro de dépôt: 03775466.0
(22) Date de dépôt: 03.10.2003
(51) Int. Cl.: A61K 9/20, A61K 9/50

(54) **SPHEROIDES PROCEDE DE PREPARATION ET COMPOSITIONS PHARMACEUTIQUES**
SPHÄROIDE, DEREN HERSTELLUNGSVERFAHREN UND SPHÄROIDENHALTIGE ARZNEIMITTEL
SPHEROIDS, PREPARATION METHOD THEREOF AND PHARMACEUTICAL COMPOSITIONS

(30) Priorité: 04.10.2002 FR 0212333
(43) Date de publication de la demande: 06.07.2005
(73) Titulaire: ETHYPHARM, 78550 Houdan (FR)
(72) Inventeur: CHENEVIER, Philippe, Montréal, Quebec H3T 1F7 (CA); MARECHAL, Dominique, LAVAL, QUEBEC H7H 2W8 (CA)
(74) Mandataire: Ahner, Francis
(86) Numéro de dépôt international: PCT/FR2003/002909
(87) Numéro de publication internationale: WO 2004/030657

(56) Documents cités:
- EP-A- 0 195 476
- EP-A- 0 217 778
- WO-A-02/19991
- WO-A-96/01624
- WO-A-99/26608
- WO-A-99/26626

## Description

La présente invention concerne des sphéroïdes gastro-résistants enrobés d'un film souple et déformable, et les comprimés multiparticulaires comprenant lesdits sphéroïdes.

La présente invention s'étend en outre au procédé de préparation de tels sphéroïdes entériques et aux comprimés multiparticulaires contenant ces sphéroïdes.

La présente invention porte enfin sur une nouvelle utilisation de mélange de mono-, di- et triglycérides et de mono- et diester de polyéthylèneglycol.

On entend par sphéroïdes des unités sphériques dont la taille peut varier de 0,1 mm à 2 mm, de préférence de 0,1 mm à 2 mm.

L'enrobage entérique permet aux noyau comprenant le principe actif de rester intact pendant le temps de séjour dans l'estomac, d'environ deux heures, dans un milieu dont le pH est compris approximativement entre 1 et 3. Une fois dans le petit intestin, comprenant le duodénum, le jéjunum et l'iléon, l'enrobage entérique va se solubiliser rapidement dans un milieu dont le pH est supérieur à 4,5 et augmente progressivement jusqu'à un pH d'environ 7,2 dans sa partie distale.

L'art antérieur comprend de nombreux exemples de comprimés multiparticulaires comprenant des granules enrobés.

Cependant, il a été montré que les agents filmogènes utilisés habituellement pour enrober les granules, ne peuvent pas absorber normalement les contraintes mécaniques appliquées lors de la compression (International Journal of Pharmaceutics, n°143, 13-23, 1996).

La compression de granules enrobés est une opération délicate en modifiant la structure du film d'enrobage par l'apparition de fissures ou par rupture, elle peut provoquer la perte partielle ou totale des propriétés du film.

La fissuration des granules modifie irréversiblement le profil de libération du ou des principes actifs qu'ils contiennent.

Les films constitués seulement de polymères ou copolymères entériques tels que l'Eudragit^{®}L30D ont des propriétés mécaniques très médiocres, telles qu'ils ne résistent pas à la compression.

Une alternative peut consister en l'ajout dans le film entérique d'autres polymères dotés de propriétés mécaniques les rendant aptes à être comprimés.

Le document Drugs made in Germany 37, n°2 (1994), p.53 enseigne qu'il est possible de combiner l'Eudragit^{®}L30D et l'Eudragit^{®}NE30D, pour obtenir des comprimés multiparticulaires comprenant lesdites particules enrobées. Cependant l'exemple III montre que cette approche ne marche pas pour tous les principes actifs.

De façon à conserver les caractéristiques du film d'enrobage des granules après compression, une autre solution consiste à diluer les granules avec des substances auxiliaires, dont le rôle est d'absorber les contraintes physiques de la compression (agents liants) et de permettre la désagrégation du comprimé (agents désintégrants) en milieu liquide, i.e. en solution aqueuse ou dans le fluide digestif.

La demande internationale WO 96/01624 (ASTRA ZENECA) porte sur un comprimé multiparticulaire comprenant des microgranules gastro-résistants, dont la proportion desdits granules au sein des comprimés est inférieure à 75% en poids, de préférence inférieur à 60% en poids, par rapport au poids total du comprimé le complément étant un diluant protégeant les granules. Dans les exemples de la demande, la proportion de granules entériques ne dépasse pas 33% du poids total du comprimé.

L'ajout de ces substances auxiliaires rend ces formes peu adaptées lorsque le dosage est important, complique le procédé en rajoutant des étapes de mélange, et augmente également le coût unitaire de la formulation

La demande internationale WO 02/19991 (ROHM) porte sur un comprimé multiparticulaire et des microgranules gastro-résistants, lesdits microgranules comprenant un enrobage entérique d'un copolymère d'acide méthycrylique et le propylène glycol. La proportion desdits granules au sein des comprimés est comprise entre 35 et 90%, de préférence 40 à 70% en poids, par rapport au poids total du comprimé, le complément étant un liant.

La demande internationale WO 99/26608 de la Demanderesse, porte sur des sphéroïdes contenant un ou plusieurs principes actifs, compressibles directement, sans l'ajout d'une partie substantielle d'une substance auxiliaire.

Ces sphéroïdes sont constitués d'un noyau contenant le principe actif, celui-ci étant recouvert d'une première couche contenant au moins un excipient thermoplastique, dont la consistance est pâteuse à semi-solide à une température de l'ordre de 20°C, et dont la température de fusion est comprise entre environ 25°C et environ 100°C, le sphéroïde résultant étant lui-même enrobée d'un film souple et déformable, à base d'un matériau polymérique.

Bien que particulièrement adaptés pour la préparation de formes gastro-résistantes, ces sphéroïdes présentent l'inconvénient d'être constitués par une pluralité de couches successives de compositions différentes impliquant un procédé de préparation long et contraignant, et d'utiliser des excipients thermoplastiques dont la consistance pâteuse à semi-solide à 20°C rend leur manipulation peu aisée.

Il est donc particulièrement intéressant d'obtenir des sphéroïdes gastro-résistants dépourvus de toute couche protectrice constituée d'excipients thermoplastiques, mais résistants néanmoins aux contraintes de la compression de telle sorte qu'il soit possible de conserver la propriété de gastro-résistance, et ce, sans avoir besoin d'ajouter d'importantes quantités d'agents auxiliaires.

La Demanderesse s'est aperçu que contrairement à ce qu'enseigne l'art antérieur, il est tout à fait possible d'améliorer suffisamment les propriétés mécaniques des films entériques, pour pouvoir réaliser des sphéroïdes enrobés par un tel film, suffisamment souple et déformable, pour être comprimés directement sans ajout de plus d'environ 5% en poids de substances auxiliaires.

L'utilisation de Gélucire® dans des enrobages à base de polymères entériques, permet d'améliorer de façon surprenante, leurs propriétés mécaniques, de telle façon que les sphéroïdes enrobés par cette composition enrobante peuvent ensuite être comprimés directement sans ajout de plus d'environ 5% en poids de substances auxiliaires.

L'objet de la présente invention est un sphéroïde gastro-résistant directement compressible , caractérisé en ce qu'il comprend :
➢ un noyau contenant un ou plusieurs principe(s) actif(s) et au moins un agent liant, directement enrobé par
➢ un film souple et déformable, comprenant un polymère entérique et un mélange de glycérides polyglycosylés saturés et/ou insaturés dont les acides gras comprennent au moins 8 atomes de carbone,
➢ une couche externe hydrodispersible contenant au moins un agent désintégrant.

Par "directement compressible", on comprend que les sphéroïdes peuvent être comprimés sous la forme de comprimés multiparticulaires sans qu'il soit nécessaire d'ajouter plus d'environ 5% de substances auxiliaires au moment de la compression.

Le noyau contient un ou plusieurs principe(s) actif(s) choisi(s) parmi ceux du groupe comprenant les sédatifs gastrointestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Les principes actifs particulièrement préférés dans cette application sont les principes actifs labiles en milieu acide, nécessitant une gastro-protection pour une administration par voie orale, par exemple les inhibiteurs de la pompe à protons, tels que l'oméprazole, le lansoprazole, le pantoprazole, le pariprazole, le léminoprazole et le rabéprazole sous leur forme racémique ou d'énantiomères pures, se présentant eux-mêmes, sous forme de base ou de sels alcalins.

D'autres principes actifs préférés sont les principes actifs irritant pour la muqueuse stomacale, dont les effets ulcérogènes nécessitent une administration retardée, tels que les anti-inflammatoires non stéroïdiens, par exemple le diclofenac, les antibiotiques tels de l'érythromycine et ses dérivés, la doxycycline. Enfin, cette forme est tout a fait intéressante pour les principes actifs présentant un site d'absorption particulier nécessitant une libération retardée.

Le ou les principe(s) actif(s) sont appliqués par montage à la surface d'un noyau neutre d'un mélange de saccharose et d'amidon, ou de cellulose microcristalline ou encore selon un procédé alternatif, sont dispersés dans la masse du noyau, par granulation sèche, humide ou à chaud, ou par extrusion-sphéronisation.

Le principe actif, initialement sous forme de poudre ou de microcristaux est utilisé sous forme de solution ou suspension dans un solvant aqueux ou organique pour le montage sur neutres, et généralement à l'état sec dans les autres cas.

Selon la présente invention, les noyaux comprennent également un liant.

Le liant est choisi dans le groupe comprenant notamment des polymères cellulosiques, des polymères acryliques, des povidones, des copovidones, des polyvinylalcools, l'acide alginique, l'alginate de sodium, l'amidon, l'amidon prégélatinisé, les sucroses et leurs dérivés, la gomme guar, les polyéthylèneglycols et leurs mélanges.

Parmi les polymères cellulosiques, on choisira avantageusement l'éthycellulose, l'hydroxypropylcellulose et l'hydroxypropylméthylcellulose, seuls ou en mélange.

Parmi les polymères acryliques, on choisit avantageusement le copolymère ammonio-méthacrylate, les polymères et copolymères d'acide acrylique et méthacryliques, les polyacrylates et les polyméthacrylate, utilisés seuls ou en mélange.

Le liant est présent dans des proportions pouvant aller jusqu'à environ 50 % en poids, de préférence jusqu'à environ 20% en poids par rapport au poids des noyaux non enrobés.

Son rôle est de fixer le principe actif sur les neutres sans perte de matière, ou de « coller » la poudre ou les microcristaux de principe actif et les autres excipients, afin de donner des particules homogènes en principe actif dont la taille rendra plus aisée l'opération d'enrobage.

Le noyau comprend de façon optionnelle, un diluant et un agent antistatique.

Le diluant peut être choisi dans le groupe comprenant notamment les dérivés cellulosiques et préférentiellement la cellulose microcristalline, les amidons seuls, le lactose, les polyols, préférentiellement le mannitol, les minéraux, préférentiellement le phosphate dicalcique.

Le diluant est présent dans des proportions pouvant aller jusqu'à environ 95% en poids, de préférence jusqu'à environ 50% en poids par rapport au poids des particules non enrobés.

Son rôle est d'augmenter la masse totale de particules à enrober, et d'obtenir une population de particules de taille homogène.

L'agent antistatique peut être choisi dans le groupe comprenant notamment la silice colloïdale, par exemple celle commercialisée sous la marque Aérosil^{®}, préférentiellement la silice précipitée, par exemple celle commercialisée sous le nom Syloïd^{®} FP244, le talc micronisé ou non, et leurs mélanges.

L'agent antistatique est présent dans des proportions pouvant aller jusqu'à environ 10% en poids, de préférence jusqu'à environ 3% en poids par rapport au poids des particules non enrobés et améliore la fluidisation de la matière lors de l'utilisation d'un lit d'air fluidisé, en particulier dans le cas d'une granulation de poudre.

Une couche polymérique optionnelle peut être appliquée entre le noyau et le film polymérique souple et déformable pour isoler le noyau actif de la couche de polymère permettant de renforcer la gastro-protection du principe actif.

Dans ce cas, le polymère est choisi parmi les mêmes polymères que ceux utilisés comme liant. Il peut être identique ou différent de celui utilisé comme liant dans le noyau actif.

La quantité de polymère appliquée est comprise entre 1 et 10% en gain de poids par rapport à la masse de noyaux actifs mise en oeuvre, de préférence entre 2 et 5%.

Le noyau comprenant le principe actif est ensuite enrobé par un film souple et déformable permettant d'assurer la gastro-protection du principe actif et constitué d'un polymère entérique et d'au moins un plastifiant.

Le polymère entérique est choisi dans le groupe comprenant l'acétatophtalate de cellulose, l'hydroxyproprylméthylcellulose phtalate, hydroxyproprylméthylcellulose succinate phtalate, polyvinyl acétate phtalate, cellulose acétate trimellitate, carboxyméthylcellulose, shellac ou tout autre polymère entérique, utilisés, seuls en mélange ou combinés séparément. Le polymère préféré est le copolymère d'acide méthacrylique, commercialisé sous le nom de marque Eudragit^{®}L100 ou Eudragit^{®}L30D.

La composition d'enrobage est appliqué par pulvérisation pour former un film continu recouvrant la totalité de la surface de chaque particule, et ce quel que soit son état de surface, en quantité suffisante pour assurer la gastro-protection du principe actif.

La gastro-protection est déterminée par un test en deux étapes, consistant à mesurer le profil de dissolution de la forme enrobée, tel que lorsque celle-ci est placée dans un milieu de dissolution de pH égal à 1,2, le pourcentage de principe actif libéré après 120 minutes est inférieur à 10% en poids, puis lorsque qu'après modification du pH du milieu d'une valeur de 1,2 à un pH de valeur 6,8, le pourcentage de principe actif libéré après 60 minutes à ce pH, est au moins égale à 80%, exprimé en poids.

Le polymère entérique est présent dans des proportions pouvant aller jusqu'à environ 50%, de préférence jusqu'à environ 20%, calculé en gain de poids par rapport à la masse de noyaux à enrober.

Le solvant choisi pour pulvériser le polymère entérique peut être l'eau, un solvant organique, tel que l'éthanol, l'isopropanol, l'acétone, ou un mélange de solvants.

Le polymère se trouve alors sous forme de solution, suspension, ou dispersion colloïdale, dans le solvant ou mélange de solvants. Il est de préférence sous forme de dispersion colloïdale dans l'eau.

De façon optionnelle, ce polymère peut être mélangé à un second polymère ou copolymère, qui peut lui-même être soluble ou insoluble, en particulier un copolymère neutre d'esters d'acides acrylique et méthacrylique, commercialisé sous le nom de marque Eudragit^{®}NE30D.

L'ajout d'un second polymère dans la composition enrobante permet d'améliorer les propriétés mécanique du film entérique résultant de ce mélange. Dans ce cas, le second polymère est ajouté dans une quantité d'au plus 100% calculé en poids sec de polymère par rapport au poids sec du polymère entérique, de préférence dans un rapport compris entre 10 et 30%.

Le mélange de glycérides polyglycosylés saturés et/ou insaturés dont les acides gras comprennent au moins 8 atomes de carbone, est en particulier un mélange de mono-, di- et triglycérides et de mono- et diester de polyéthylèneglycol (PEG), de poids moléculaire compris entre 200 et 1500, éventuellement de glycérol et de PEG libre.

Ledit mélange est par exemple commercialisé sous le nom de marque Gélucire®.

Les acides gras du mélange de glycérides polyglycosylés saturés et/ou insaturés comprennent préférentiellement de 8 à 18 atomes de carbones (C8-C18).

Par C8-C18, on désigne des mélanges en proportions significatives et variables des acides caprylique (C8), caprique (C10), laurique (C12), myristique (C14), palmitique (C16) et stéarique (C18), lorsque ces acides sont saturés et les acides insaturés correspondants en (C8-C18). Les proportions de ces acides gras peuvent varier en fonctions des huiles de départ.

Parmi les Gélucires^{®}, on préfère la Gélucire® 50/13 qui comprend ainsi de façon prédominante, l'acide palmitostéarique (C16-C18) et caractérisée par un point de fusion compris entre 46.0 et 51.0°C et une valeur de balance hydrophile/lipophile (HLB) égale à 13.

La proportion totale du mélange de glycérides polyglycosylés saturés et/ou insaturés est d'au plus 40%, de préférence entre 10 à 30%, exprimé en poids par rapport au poids sec de polymère.

La fonction du mélange de glycérides polyglycosylés saturés et/ou insaturés est d'abaisser la température de transition vitreuse du film et d'améliorer les propriétés mécaniques du film polymérique d'enrobage, en particulier de le rendre souple et déformable.

De façon optionnelle, le film d'enrobage comprend également un plastifiant choisi dans le groupe comprenant le triéthyl citrate, l'acétyltributyl citrate, la triacétine, le tributyl citrate, le diéthylphtalate, les polyéthylènes glycols, les polysorbates, les glycérides mono-et diacétylés.

Le plastifiant est utilisé dans une proportion totale d'au plus 40%, de préférence entre 10 à 30%, exprimé en poids par rapport au poids sec de polymère.

La fonction du plastifiant est d'abaisser la température de transition vitreuse du film.

La composition enrobante comprend également de façon optionnelle, un agent tensioactif, un agent antistatique, un lubrifiant.

L'agent tensioactif est choisi parmi les tensioactifs anioniques, cationiques, non ioniques ou amphotères.

L'agent antistatique, est utilisé comme pour éviter les problèmes liés à l'électricité statique. Il est dans le groupe comprenant le talc micronisé ou non micronisé, la silice colloïdale (Aerosil^{®}200), la silice traitée (Aerosil^{®} R972), ou la silice précipitée (Syloïd^{®} FP244) et leurs mélanges.

L'agent antistatique est utilisé dans une proportion d'au plus environ 10% en poids, de préférence entre 0 et 3%, de préférence inférieure à environ 1% en poids.

Le lubrifiant est choisi dans le groupe comprenant le stéarate de magnésium, l'acide stéarique, sodium stearyl fumarate, les polyoxyéthylèneglycols micronisés, le benzoate de sodium et leurs mélanges.

La granulométrie de ces sphéroïdes permet leur utilisation dans la fabrication de comprimés multiparticulaires.

De façon avantageuse, les sphéroïdes ont un diamètre compris entre 0,1 mm et 2 mm, de préférence entre 0,3 mm et 1 mm.

La taille est déterminée par les méthodes conventionnelle, par exemple à l'aide d'un jeu de tamis de maille calibré, ou par diffraction d'un laser.

Les sphéroïdes selon l'invention sont avantageusement enrobés d'une couche externe hydrodispersible.

Cette couche assure la cohésion des sphéroïdes entre eux au moment de la compression et ainsi la dureté du comprimé, et permet le délitement en milieu aqueux du comprimé obtenu.

La couche externe hydrodispersible est constituée d'au moins un désintégrant.

Le désintégrant est choisi dans le groupe comprenant notamment la carboxyméthylcellulose sodique réticulée désignée dans le métier par le terme croscarmellose, la crospovidone, le carboxyméthyl amidon sodique et leurs mélanges.

Elle peut comprendre de façon optionnelle un liant sélectionné parmi ceux utilisés pour l'étape de montage, et des substances auxiliaires hydrosolubles telles que des polyols, en particulier le mannitol.

L'invention porte également sur le procédé de préparation des sphéroïdes gastro-résistants directement compressibles.

Le procédé conforme à l'invention comprend les étapes suivantes :
➢ préparation d'un noyau contenant un ou plusieurs principe(s) actif(s) et au moins un liant ;
➢ enrobage des noyaux ainsi obtenus par pulvérisation de la composition d'enrobage contenant un polymère entérique, un mélange de glycérides polyglycosylés saturés et/ou insaturés dont les acides gras comprennent au moins 8 atomes de carbone, préférentiellement de 8 à 18 atomes de carbones (C8-C18)
➢ enrobage des sphéroïdes gastro-résistants par une couche externe hydrodispersible contenant au moins un agent désintégrant, et séchage des sphéroïdes.

Selon ce mode de mise en oeuvre, les étapes peuvent être réalisées au sein d'appareils différents ou du même appareil.

Le noyau comprenant le principe actif peut-être obtenu par granulation, par montage sur neutre, ou encore par extrusion-sphéronisation.

Dans un premier mode de réalisation, les noyaux comprenant le principe actif, sont préparés par granulation selon les étapes suivantes :
➢ mélange à sec du principe actif sous forme de poudre ou de microcristaux, éventuellement avec l'agent diluant et un agent antistatique,
➢ granulation du mélange obtenu par pulvérisation d'une solution de l'agent liant,
➢ séchage.

Pour la granulation, un granulateur haute énergie, un mélangeur planétaire ou un lit d'air fluidisé sont avantageusement utilisés.

Dans un second mode de réalisation des noyaux comprenant le principe actif, ceux-ci sont préparés par montage sur neutres selon les étapes suivantes :
➢ pulvérisation sur des neutres d'une solution ou une suspension du principe actif, contenant le liant solubilisé et de façon optionnelle un lubrifiant, un agent antistatique
➢ séchage,

La préparation de montage peut se présenter, selon les cas, sous forme de suspension en milieux aqueux ou organiques, sous forme de solutions, sous forme d'émulsions ou à l'état fondu.

Dans une première variante du procédé de montage, le principe actif est incorporé à la préparation de montage.

Selon une autre variante du procédé de montage, le principe actif est appliqué par poudrage sur les noyaux neutres préalablement mouillés avec la préparation de montage.

Toutes les étapes du procédé conforme à l'invention peuvent être réalisées dans une turbine à dragéification une turbine perforée ou en lit d'air fluidisé.

Selon un troisième mode de réalisation, les noyaux comprenant le principe actif sont préparés par extrusion-sphéronisation.

Le principe actif est alors mélangé à la masse d'excipient. Le mélange est humidifié pour assurer une extrusion satisfaisante, les extrudats ainsi obtenus sont calibrés et sphéronisés.

Les noyaux ainsi obtenus sont ensuite enrobés à l'aide d'une composition contenant un polymère entérique filmogène, un plastifiant, et de façon optionnelle, un agent tensioactif, un agent antistatique, un lubrifiant.

La composition enrobante est pulvérisée sous forme de solution, de suspension ou de dispersion colloïdale de ce polymère dans un solvant organique ou aqueux, puis séchée.

La couche externe hydrodispersible est appliquée avec l'une des méthodes précédentes, cependant que l'alcool isopropylique est le solvant utilisé de préférence.

Dans un mode de réalisation préféré du procédé de préparation des sphéroïdes gastro-résistants, toutes les étapes de préparation du noyau actif et d'enrobage sont effectuées en lit d'air fluidisé.

Le lit d'air fluidisé est équipé d'une buse de pulvérisation dont la position et l'orientation de pulvérisation peuvent être choisie. Le mode de pulvérisation est dit "top spray", "bottom spray" ou "tangential spray", selon la terminologie habituelle de l'homme du métier.

Le choix du mode de pulvérisation permet de maîtriser la cinétique de croissance des particules et d'éviter des phénomènes de collage, liés à la nature du principe actif, à la composition liante ou enrobante pulvérisée, et aux divers paramètres du procédé (température, pression d'air par exemple, débit de solution).

La présente invention a également pour objet les comprimés multiparticulaires contenant les sphéroïdes directement compressibles décrits précédemment, et ne comprenant pas plus d'environ 5% en poids au total d'une substance auxiliaire, tel qu'un lubrifiant, un agent antistatique et/ou un agent perméabilisant.

Le lubrifiant est choisi dans le groupe comprenant le stéarate de magnésium, l'acide stéarique, sodium stéaryl fumarate, les polyoxyéthylèneglycols micronisés, le benzoate de sodium et leurs mélanges.

L'agent antistatique est utilisé pour éviter les problèmes liés à l'électricité statique. Il est dans le groupe comprenant le talc micronisé ou non micronisé, la silice colloïdale (Aerosil^{®}200), la silice traitée (Aerosil^{®} R972) ou la silice précipitée (Syloïd^{®} FP244) et leurs mélanges.

L'agent perméabilisant est choisi dans le groupe comprenant notamment des silices ayant une grande affinité pour les solvants aqueux, telles que la silice précipitée plus connue sous le nom de marque Syloïd^{®}, les maltodextrines, les β-cyclodextrines et leurs mélanges.

L'agent perméabilisant permet la création d'un réseau hydrophile qui contribue ainsi à une meilleure désagrégation du comprimé.

Les comprimés multiparticulaires selon l'invention se désintègrent en solution en moins de 60 minutes et restituent des sphéroïdes indépendants, de sorte que le profil de libération du comprimé et des sphéroïdes qui le constituent sont pratiquement équivalents.

En effet, les comprimés selon l'invention permettent la délivrance de sphéroïdes sans que le profil de libération du ou des principe(s) actif(s) qu'ils contiennent ne soit altéré sous l'effet de la compression.

Les comprimés selon l'invention peuvent être composés uniquement des sphéroïdes selon l'invention ou d'un mélange de sphéroïdes contenant un ou plusieurs principes actifs et de sphéroïdes placebos, c'est-à-dire des sphéroïdes conformes à la présente invention sans principe actif.

Les comprimés selon l'invention peuvent faire l'objet d'un enrobage final de protection ou de coloration.

L'invention porte également sur le procédé de préparation des comprimés multiparticulaires comprenant les sphéroïdes.

Le procédé conforme à l'invention comprend les étapes suivantes :
➢ mélange des sphéroïdes gastro-résistants avec pas plus de 5% en poids au total d'une ou plusieurs substances auxiliaires, tel qu'un lubrifiant, un agent antistatique et/ou un agent perméabilisant,
➢ compression du mélange pour obtenir une forme unitaire.

La compression des sphéroïdes peut être réalisée sur une machine à comprimer alternative ou rotative.

Les contraintes exercées sur les sphéroïdes, lors de l'étape de compression, peuvent varier de 5 kN à 50 kN et de préférence entre 5 kN et 15 kN.

La dureté de ces comprimés est de préférence comprise entre 1 et 10kp, plus préférentiellement entre 1 et 5kp, mesurée selon la méthode de la Pharmacopée Européenne (2.9.8), 1kp étant égal à 9,8N.

De préférence, on adapte la dureté du comprimé multiparticulaire de façon à obtenir une friabilité, mesurée selon la méthode de la Pharmacopée Européenne, inférieure à 2%

Le temps de désagrégation des comprimés en milieu aqueux à 37°C est inférieur à 60 minutes

Les comprimés peuvent avoir un diamètre compris entre 6mm et 17 mm. Leur forme peut être ronde, ovale, oblongue, de surface plate ou concave, et présenter des gravures ou des barres des sécabilité.

Les comprimés selon l'invention ont de préférence une masse comprise entre 0,1 g et 2 g.

L'invention sera mieux comprise au moyen des exemples de réalisation des sphéroïdes gastro-résistants et des comprimés multiparticulaires conformes à l'invention. Ces exemples sont donnés uniquement à titre d'illustrations et de modes de réalisation avantageux de l'invention et n'en constituent nullement une limitation.

### METHODES ANALYTIQUES [USP - 724]

Test de gastro-résistance

On détermine le profil de dissolution des sphéroïdes gastro-résistants dans les conditions suivantes :
➢ Appareil : USP type II
➢ vitesse de pales : 100 rpm
➢ volume : 750 ml pH 1,2 et 1000 ml pH 6,8
➢ température : 37.0°C ±0.5°C
➢ détection: spectrophotométrie UV directe à 272 nm
➢ Milieu de dissolution : HCl 0,1 N (pH = 1,2) pendant 120 minutes (t0 à t 120mn), puis pH 6,8 pendant 60mn (t =121mn à t=180mn)

### EXEMPLE 1:

### Montage du principe actif en Lit d'Air Fluidisé

Dans un Lit d'Air Fluidisé de type GPCG-3, équipé d'une buse Würster ("bottom spray"), on pulvérise sur 318,5 grammes de noyaux neutres une suspension contenant 636,9 grammes de théophylline et du PEG400 comme liant, 30% en poids par rapport à la théophylline.

### Enrobage

1000 grammes de granules obtenus après l'étape de montage décrite précédemment sont enrobés dans un lit d'air fluidisé GLATT GPCG-3 équipé par un insert Würster par pulvérisation d'une dispersion aqueuse d'Eudragit^{®}L30D, comprenant 30% en poids de triéthylcitrate (TEC), calculé par rapport au poids sec de polymère.

On applique sur les granules de théophylline une quantité totale d'Eudragit^{®}L30D correspondant à 30% calculé en gain de poids par rapport à la masse de granules non enrobés de départ

Les granules enrobés G1 issus de l'étape d'enrobage présentent le profil de dissolution suivant :

**Tableau 1**

| Théophyline libérée % (w/w) | |
|---|---|
| Temps | G1 |
| **pH 1,2** | |
| 120 mn | 7 |
| **→ pH 6,8** | |
| 135 mn | 81 |
| 150 mn | 81 |
| 180 mn | 81 |

### Conclusion

Les sphéroïdes présentent un profil de dissolution satisfaisant aux spécifications du test de gastro-résistance.

### EXEMPLE 2

### Surenrobage

Les granules G1 de l'exemple 1 sont enrobés d'une couche externe constituée d'un agent liant mais dépourvu d'agent désintégrant.

On pulvérise sur les granules G1 une solution aqueuse contenant soit du PEG 4000, soit un mélange de PEG 4000 et d'HPMC 603 dans une proportion 20/80, comprenant en outre 20% en poids de talc micronisé, calculé par rapport au poids sec total de polymère.

Chaque sous-lot de granules est comprimé séparément sur une presse MANESTY F3 équipée d'un poinçon rond, convexe de diamètre 10mm de façon à obtenir une valeur de friabilité inférieure à 2% en poids.

Après 60 minutes, les comprimés ainsi obtenus ne sont pas désintégrés.

### Conclusion :

La couche externe dispersible dépourvue d'un désintégrant ne permet pas la désagrégation du comprimé conformément aux spécifications.

### EXEMPLE 3

### Surenrobage

Les granules G1 de l'exemple 1 sont enrobés d'une couche hydrodispersible comprenant un agent désintégrant, l'Ac-Di-Sol^{®}.

Le l'Ac-Di-Sol^{®} mélangé avec du mannitol 25 dans un rapport 50/50, est appliqué sur les granules G1 par poudrage dans une turbine classique, en utilisant une solution liante comprenant de la polyvinylpyrrolidone (PVP) K29, en solution à 10% dans de l'alcool isopropylique.

On applique sur les granules une quantité d'Ac-Di-Sol^{®}, correspondant à 20% en poids, calculé par rapport à la masse de granules G1 de départ.

Les granules G1/1 ainsi préparés présentent le profil de dissolution suivant :

**Tableau 2**

| Théophylline libérée % (w/w) | |
|---|---|
| Temps | G1/1 |
| **pH 1,2** | |
| 120 mn | 7 |
| **→ pH 6, 8** | |
| 135 mn | 79 |
| 150 mn | 79 |
| 180 mn | 80 |

### Compression

Les granules obtenus à l'étape précédente G1/1 sont comprimés sur une presse MANESTY F3 équipée d'un poinçon rond, convexe de diamètre 10mm de façon à obtenir un dosage unitaire en théophylline d'environ 50 mg.

Les comprimés (C1/1) ainsi obtenus présentent les caractéristiques suivantes :

**Tableau 3**

| | C1/1 |
|---|---|
| Poids (mg) | 426 |
| Dureté (kP) | 2,7 |
| Friabilité (%) | 0,21 |
| Désagrégation (mn) | 50 |
| Théophylline libérée | |
| % (w/w) | |
| **pH 1,2** | |
| 120 mn | 51 |
| **→ pH 6, 8** | |
| 135 mn | 87 |
| 150 mn | 88 |
| 180 mn | 88 |

### Conclusion :

La couche hydrodispersible comprenant l'Ac-Di-Sol^{®} permet la désagrégation du comprimé en moins de 60 minutes, cependant que la compression des granules provoquent la rupture du film polymérique et la perte de la gastro-protection.

Le comprimé ne satisfait pas aux spécifications du test de gastro-résistance.

### EXEMPLE 4

### Surenrobage

Les granules G1 de l'exemple 1 sont enrobés d'une couche hydrodispersible comprenant un agent désintégrant, le Kollidon®CLM.

Le Kollidon®CLM, mélangé avec du mannitol 25 dans un rapport 50/50, est appliqué sur les granules G1 par poudrage dans une turbine classique, en utilisant une solution liante comprenant de la PVP K29, en solution à 10% en poids dans de l'alcool isopropylique.

On applique sur les granules une quantité de Kollidon®CLM correspondant à 20% en poids, calculé par rapport à la masse de granules G1 de départ.

Les granules ainsi préparés (G1/2) présentent le profil de dissolution suivant :

**Tableau 4**

| Théophylline libérée %(w/w) | |
|---|---|
| Temps | G1/2 |
| **pH 1,2** | |
| 120 mn | 5 |
| **→ pH 6,8** | |
| 135 mn | 82 |
| 150 mn | 82 |
| 180 mn | 82 |

### Compression

Les granules obtenus à l'étape précédente sont comprimés sur une presse MANESTY F3 équipée d'un poinçon rond, convexe de diamètre 10mm de façon à obtenir un dosage unitaire en théophylline d'environ 70 mg.

Les comprimés (C1/2) ainsi obtenus présentent les caractéristiques suivantes :

**Tableau 5**

| | C1/2 |
|---|---|
| Poids (mg) | 409 |
| Dureté (kP) | 2,5 |
| Friabilité (%) | 0,51 |
| Désagrégation (mn) | 32 |
| Théophylline libérée | |
| % (w/w) | |
| **pH 1,2** | |
| 120 mn | 41 |
| **→ pH 6, 8** | |
| 135 mn | 80 |
| 150 mn | 81 |
| 180 mn | 82 |

### Conclusion :

La couche hydrodispersible comprenant l'agent désintégrant permet la désagrégation du comprimé en moins de 60 minutes, cependant que la compression des granules provoquent la rupture du film polymérique et la perte de la gastro-protection.

Le comprimé ne satisfait pas aux spécifications du test de gastro-résistance.

### EXEMPLE 5

### Montage du principe actif en Lit d'Air Fluidisé (RD239)

Dans un Lit d'Air Fluidisé de type GLATTGPCG-3, équipé d'une buse Würster, on pulvérise sur 318.5 grammes de noyaux neutres une suspension contenant 636.9 grammes de théophylline et un mélange PVPK29/Eudragit^{®}RS100 comme liants, représentant au total 50% en poids par rapport à la théophylline.

### Enrobage

1000 grammes de granules obtenus après l'étape de montage sont enrobés dans un lit d'air fluidisé GLATT GPCG-3 équipé par un insert Würster par pulvérisation d'une dispersion aqueuse d'un mélange Eudragit®L30D/Gélucire®50/13 dans un rapport 75/25, comprenant en outre 10% en poids de triéthylcitrate (TEC), calculé par rapport au poids sec d'Eudragit^{®}L30D.

On applique sur les granules une quantité totale du mélange Eudragit®L30D/Gélucire®50/13 correspondant à 50% calculé en gain de poids par rapport à la masse de granules non enrobés de départ.

Les granules enrobés G2 ainsi enrobés présentent le profil de dissolution suivant :

**Tableau 6**

| Théophylline libérée % (w/w) | |
|---|---|
| Temps | G2 |
| **pH 1,2** | |
| 120 mn | 3 |
| **→ pH 6,8** | |
| 135 mn | 92 |
| 150 mn | 109 |
| 180 mn | 110 |

### Surenrobage

Les granules G2 obtenus à l'étape précédente sont enrobés d'une couche hydrodispersible comprenant du Kollidon®CLM.

Le Kollidon®CLM, mélangé avec du mannitol 25 dans un rapport 50/50, est appliqué sur les granules par poudrage dans une turbine classique, en utilisant une solution liante comprenant de la PVP K29, en solution à 10% dans de l'alcool isopropylique.

On applique sur les granules une quantité de Kollidon®CLM correspondant à 20% en poids, calculé par rapport à la masse de granules G2 de départ.

### Compression

Les granules enrobés obtenus à l'étape précédente (G2/1) sont comprimés sur une presse MANESTY F3 équipée d'un poinçon rond, convexe de diamètre 12mm de façon à obtenir un dosage unitaire en théophylline d'environ 150 mg.

Les comprimés ainsi obtenus (C2/1) présentent les caractéristiques suivantes :

**Tableau 7**

| | C2/1 |
|---|---|
| Poids (mg) | 400 |
| Dureté (kP) | 6.0 |
| Friabilité (%) | Nd |
| Désagrégation (mn) | 26 |
| Théophylline libérée | |
| % (w/w) | |
| **pH 1,2** | |
| 120 mn | 3 |
| **→ pH 6, 8** | |
| 135 mn | 64 |
| 150 mn | 89 |
| 180 mn | 92 |

### Conclusion

Les sphéroïdes satisfont aux spécifications du test de gastro-résistance.

Le comprimé satisfait aux spécifications du test de gastro-résistance et de désintégration.

## Revendications

1. Sphéroïde gastro-résistant directement compressible, **caractérisé en ce qu'**il comprend :
➢ un noyau contenant un ou plusieurs principe(s) actif(s) directement enrobé par,
➢ un film souple et déformable, comprenant un polymère entérique et un mélange de glycérides polyglycosylés saturés et/ou insaturés dont les acides gras comprennent au moins 8 atomes de carbone,
➢ une couche externe hydrodispersible contenant au moins un agent désintégrant.

2. Sphéroïde selon la revendication 1 **caractérisé en ce que** le noyau contient un ou plusieurs principe(s) actif(s) choisi(s) parmi ceux du groupe comprenant les sédatifs gastrointestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

3. Sphéroïde selon l'une des revendications 1 et 2 **caractérisé en ce que** le principe actif est choisi parmi les inhibiteurs de la pompe à protons, de préférence l'oméprazole, le lansoprazole, le pantoprazole, le pariprazole, le léminoprazole, ou le rabéprazole, sous leur forme racémique ou d'énantiomères purs, se présentant eux-mêmes, sous forme de base ou de sel alcalins, les anti-inflammatoires non-stéroïdiens, de préférence le diclofénac sous forme de bases ou de sels, les antibiotiques, de préférences l'érythromicine et ses dérivés sous forme de bases ou de sels.

4. Sphéroïde selon l'une des revendications 1 à 3 **caractérisé en ce que** le liant est choisi dans le groupe comprenant des polymères cellulosiques, des polymères acryliques, des povidones, des copovidones, des polyvinylalcools, l'acide alginique, l'alginate de sodium, l'amidon, l'amidon prégélatinisé, les sucroses et leurs dérivés, la gomme guar, les polyéthylèneglycols et leurs mélanges.

5. Sphéroïde selon l'une des revendications 1 à 4 **caractérisé en ce que** le noyau comprend, de façon optionnelle, un diluant, un agent antistatique et/ou un lubrifiant.

6. Sphéroïde selon l'une des revendications 1 à 5 **caractérisé en ce que** le polymère entérique est choisi dans le groupe comprenant l'acétate phtalate de cellulose, l'hydroxyproprylméthylcellulose phtalate, l'hydroxyproprylméthylcellulose succinate phtalate, le polyvinyl acétate phtalate, le cellulose acétate trimellitate, la carboxyméthylcellulose, le shellac, utilisés seuls ou en mélange.

7. Sphéroïde selon la revendication 6 **caractérisé en ce que** le polymère entérique est un copolymère d'acide méthacrylique.

8. Sphéroïde selon l'une des revendications 1 à 7 **caractérisé en ce que** les acides gras du mélange de glycérides polyglycosylés saturés et/ou insaturés comprennent de 8 à 18 atomes de carbone (C8-C18).

9. Sphéroïde selon la revendication 8, **caractérisé en ce** ledit mélange est un mélange de mono-, di- et triglycérides et de mono- et diester de polyéthylèneglycol de poids moléculaire compris entre 200 et 1500, éventuellement de glycérol et de PEG libre comprend de façon prédominante, l'acide palmitostéarique, ledit mélange étant **caractérisée par** un point de fusion compris entre 46,0°C et 51,0°C et une valeur de balance hydrophile/lipophile (HLB) égale à 13.

10. Sphéroïde selon la revendication 8 **caractérisé en ce que** ledit mélange est la Gélucire®, en particulier la Gélucire 50/13.

11. Sphéroïde selon la revendication 1 à 10 **caractérisé en ce que** le film souple et déformable comprend de façon optionnelle un agent plastifiant choisi dans le groupe comprenant le triéthyl citrate, l'acétyltributyl citrate, la triacétine, le tributyl citrate, le diéthylphtalate, les polyethylènes glycols, les polysorbates, les glycérides mono-et diacétylés, de préférence le triéthyl citrate.

12. Sphéroïde selon la revendication 1 à 11 **caractérisé en ce que** la composition enrobante comprend de façon optionnelle, un agent tensioactif, un agent antistatique, un lubrifiant.

13. Sphéroïde selon la revendication 1 à 12 **caractérisé en ce que** le désintégrant est choisi dans le groupe comprenant la carboxyméthylcellulose sodique réticulée désignée dans le métier par le terme croscarmellose, la crospovidone, le carboxyméthyl amidon sodique et leurs mélanges.

14. Sphéroïde selon la revendication 1 à 13 **caractérisé en ce que** la couche externe dispersible comprend de façon optionnelle, un liant et une substance auxiliaire, en particulier le mannitol.

15. Procédé de préparation des sphéroïdes selon les revendications 1 à 14, **caractérisé en ce qu'**il comprend les étapes suivantes :
➢ préparation d'un noyau contenant un ou plusieurs principe(s) actif(s) et au moins un liant ;
➢ enrobage des noyaux ainsi obtenus par pulvérisation de la composition d'enrobage contenant un polymère entérique et un mélange de glycérides polyglycosylés saturés et/ou insaturés dont les acides gras comprennent au moins 8 atomes de carbone, préférentiellement de 8 à 18 atomes de carbones (C8-C18)
➢ enrobage des sphéroïdes gastro-résistants par une couche externe hydrodispersible contenant au moins un agent désintégrant, et
➢ séchage des sphéroïdes.

16. Procédé de préparation selon la revendication 15 **caractérisé en ce que** le noyau contenant le principe actif est préparé par granulation, par montage sur neutre, ou encore par extrusion-sphéronisation.

17. Procédé de préparation selon les revendications 15 et 16 **caractérisé en ce que** les sphéroïdes sont préparés en lit d'air fluidisé.

18. Comprimé multiparticulaire contenant les sphéroïdes selon l'une des revendications 1 à 17.

19. Comprimé multiparticulaire selon la revendication 18 **caractérisé en ce qu'**il ne comprend pas plus d'environ 5% en poids total d'une ou plusieurs substance(s) auxiliaire(s).

20. Comprimé multiparticulaire selon l'une des revendications 18 et 19, **caractérisé en ce que** la substance auxiliaire est un lubrifiant, un agent antistatique et/ou agent perméabilisant.

21. Comprimé multiparticulaire selon l'une des revendication 18 à 20, **caractérisé en ce qu'**il comprend un mélange de sphéroïdes contenant un ou plusieurs principes actifs et de sphéroïdes placebos.

22. Procédé de préparation des comprimés multiparticulaires selon l'une des revendications 18 à 19 **caractérisé en ce qu'**il comprend les étapes suivantes :
➢ mélange des sphéroïdes gastro-résistants avec pas plus d'environ 5% en poids total d'une ou plusieurs substances auxiliaires,
➢ compression du mélange pour obtenir une forme unitaire.

## Claims

1. Directly tabletable gastroresistant spheroid, **characterized in that** it comprises:
➢ a core comprising one or more active principles, directly coated with
> a flexible and deformable film comprising an enteric polymer and a mixture of saturated and/or unsaturated polyglycosylated glycerides whose fatty acids contain at least 8 carbon atoms,
➢ a water-dispersible outer layer comprising at least one disintegrant.

2. Spheroid according to Claim 1, **characterized in that** the core comprises one or more active principles selected from those from the group consisting of gastrointestinal sedatives, antacids, analgesics, antiinflammatories, coronary vasodilators, peripheral and cerebral vasodilators, antiinfection agents, antibiotics, antivirals, antiparasitics, anticancer agents, anxiolytics, neuroleptics, central nervous system stimulants, antidepressants, antihistamines, antidiarrheals, laxatives, nutritional supplements, immuno-depressants, hypocholesterolemics, hormones, enzymes, antispasmodics, antianginal agents, medicinal products which influence heart rate, medicinal products used in the treatment of arterial hypertension, antimigraine agents, medicinal products which influence blood clottability, antiepileptics, muscle relaxants, medicinal products used in the treatment of diabetes, medicinal products used in the treatment of thyroid dysfunctions, diuretics, anorexigenic agents, antiasthmatics, expectorants, antitussives, muco-regulators, decongestants, hypnotics, antinausea agents, hematopoietic agents, uricosuric agents, plant extracts, and contrast agents.

3. Spheroid according to either of Claims 1 and 2, **characterized in that** the active principle is selected from proton pump inhibitors, preferably omeprazole, lansoprazole, pantoprazole, pariprazole, leminoprazole or rabeprazole, in their racemic form or in the form of pure enantiomers, themselves in base form or in the form of alkali metal salts; nonsteroidal antiinflammatories, preferably diclofenac, in the form of bases or of salts; antibiotics, preferably erythromycin and its derivatives, in the form of bases or of salts.

4. Spheroid according to one of Claims 1 to 3, **characterized in that** the binder is selected from the group consisting of cellulosic polymers, acrylic polymers, povidones, copovidones, polyvinyl alcohols, alginic acid, sodium alginate, starch, pregelatinized starch, sucroses and derivatives thereof, guar gum, polyethylene glycols, and mixtures thereof.

5. Spheroid according to one of Claims 1 to 4, **characterized in that** the core optionally comprises a diluent, an antistat and/or a lubricant.

6. Spheroid according to one of Claims 1 to 5, **characterized in that** the enteric polymer is selected from the group consisting of cellulose acetate phthalate, hydroxypropylmethylcellulose phthalate, hydroxypropylmethylcellulose succinate phthalate, polyvinyl acetate phthalate, cellulose acetate trimellitate, carboxymethylcellulose and shellac, which are used alone or in a mixture.

7. Spheroid according to Claim 6, **characterized in that** the enteric polymer is a methacrylic acid copolymer.

8. Spheroid according to one of Claims 1 to 7, **characterized in that** the fatty acids of the mixture of saturated and/or unsaturated polyglycosylated glycerides contain from 8 to 18 carbon atoms (C8-C18).

9. Spheroid according to Claim 8, **characterized in that** said mixture is a mixture of mono-, di- and triglycerides and of polyethylene glycol monoester and diester, with a molecular weight of between 200 and 1500, and optionally of glycerol and of free PEG, and predominantly comprises palmitostearic acid, said mixture being **characterized by** a melting point of between 46.0°C and 51.0°C and a hydrophilic/lipophilic balance (HLB) of 13.

10. Spheroid according to Claim 8, **characterized in that** said mixture is Gélucire®, in particular Gélucire 50/13.

11. Spheroid according to Claims 1 to 10, **characterized in that** the flexible and deformable film optionally comprises a plasticizer selected from the group consisting of triethyl citrate, acetyl tributyl citrate, triacetin, tributyl citrate, diethyl phthalate, polyethylene glycols, polysorbates, and monoacetylated and diacetylated glycerides, preferably triethyl citrate.

12. Spheroid according to Claims 1 to 11, **characterized in that** the coating composition optionally comprises a surfactant, an antistat and/or a lubricant.

13. Spheroid according to Claims 1 to 12, **characterized in that** the disintegrant is selected from the group consisting of the crosslinked sodium carboxymethylcellulose denoted in the art by the term croscarmellose, crospovidone, sodium carboxymethyl starch, and mixtures thereof.

14. Spheroid according to Claims 1 to 13, **characterized in that** the dispersible outer layer optionally comprises a binder and an auxiliary substance, in particular mannitol.

15. Method of preparing the spheroids according to Claims 1 to 14, **characterized in that** it comprises the following steps:
➢ preparing a core comprising one or more active principles and at least one binder;
➢ coating the cores thus obtained by spraying the coating composition comprising an enteric polymer and a mixture of saturated and/or unsaturated polyglycosylated glycerides whose fatty acids contain at least 8 carbon atoms, preferably from 8 to 18 carbon atoms (C8-C18);
➢ coating the gastroresistant spheroids with a water-dispersible outer layer comprising at least one disintegrant; and
➢ drying the spheroids.

16. Method of preparing according to Claim 15, **characterized in that** the core comprising the active principle is prepared by granulation, by application to neutral substance, or else by extrusion with spheronization.

17. Method of preparing according to Claims 15 and 16, **characterized in that** the spheroids are prepared in a fluidized-air bed.

18. Multiparticulate tablet comprising the spheroids according to one of Claims 1 to 17.

19. Multiparticulate tablet according to Claim 18, **characterized in that** it contains not more than approximately 5% by total weight of one or more auxiliary substances.

20. Multiparticulate tablet according to either of Claims 18 and 19, **characterized in that** the auxiliary substance is a lubricant, an antistat and/or a permeabilizing agent.

21. Multiparticulate tablet according to one of Claims 18 to 20, **characterized in that** it comprises a mixture of spheroids comprising one or more active principles and of placebo spheroids.

22. Method of preparing the multiparticulate tablets according to either of Claims 18 and 19, **characterized in that** it comprises the following steps:
➢ mixing the gastroresistant spheroids with not more than approximately 5% by weight in total of one or more auxiliary substances;
➢ tableting the mixture to give a unitary form.

## Patentansprüche

1. Direkt kompressibler gastroresistenter Sphäroid, **dadurch gekennzeichnet, dass** er umfasst:
➢ einen Kern, der einen oder mehrere direkt **dadurch** umhüllte Wirkstoff/e enthält,
➢ einen elastischen und deformierbaren Film, der ein enterisches Polymer und ein Gemisch gesättigter und/oder ungesättigter polyglycosylierter Glyceride umfasst, deren Fettsäuren mindestens 8 Kohlenstoffatome umfassen,
➢ eine wasserlösliche Außenschicht, die mindestens ein Sprengmittel enthält.

2. Sphäroid nach Anspruch 1, **dadurch gekennzeichnet, dass** der Kern einen oder mehrere Wirkstoff/e enthält, der/die aus der Gruppe ausgewählt ist/sind, die die gastrointestinalen Sedativa, die Antazide, die Analgetika, die Entzündungshemmer, die Koronardilatatoren, die peripheren und zerebralen Dilatatoren, die Anti-Infektionsmittel, die Antibiotika, die antiviralen Mittel, die Mittel gegen Parasiten, die Mittel gegen Krebs, die Anxiolytika, die Neuroleptika, die Stimulantien des zentralen Nervensystems, die Antidepressiva, Antihistamine, die Mittel gegen Durchfallerkrankungen, die Abführmittel, die Nahrungsergänzungen, die Immundepressiva, die Cholesterinsenker, die Hormone, die Enzyme, die Antispasmodika, die stenokardienhemmenden Mittel, die Mittel, die den Herzrhythmus beeinflussen, die Arzneimittel, die bei der Behandlung des arteriellen Bluthochdrucks verwendet werden, die Migränemittel, die Mittel, die die Blutgerinnung beeinflussen, die Antiepileptika, die Muskelrelaxantien, die Arzneimittel, die bei der Behandlung des Diabetes verwendet werden, die Arzneimittel, die bei der Behandlung von Fehlfunktionen der Schilddrüse verwendet werden, die Diuretika, die Appetitzügler, die Asthmamittel, die auswurffördernden Mittel, die Hustenmittel, die Schleimregulierer, die Dekongestiva, die Hypnotika, die Mittel gegen Brechreiz, die Blutbildner, die Urikosurika, die Pflanzenextrakte, die Kontrastmittel umfasst.

3. Sphäroid nach einem der Ansprüche 1 und 2, **dadurch gekennzeichnet, dass** der Wirkstoff aus den Inhibitoren der Protonenpumpe ausgewählt ist, vorzugsweise dem Omeprazol, dem Lansoprazol, dem Pantoprazol, dem Pariprazol, dem Leminoprazol oder dem Rabeprazol in ihrer razemischen Form oder reiner Enantiomere, die sich selbst in Form einer alkalischen Base oder Salz darstellen, den nicht steroiden Entzündungshemmern, vorzugsweise dem Diclofenac in Form von Basen oder Salzen, den Antibiotika, vorzugsweise dem Erythromycin und seine Derivaten in Form von Basen oder Salzen.

4. Sphäroid nach einem der Ansprüche 1 bis 3, **dadurch gekennzeichnet, dass** das Bindemittel aus der Gruppe ausgewählt ist, die Zellulosepolymere, Acrylpolymere, Povidone, Copovidone, Polyvinylalkohole, die Alginsäure, das Natriumalginat, die Stärke, die Quellstärke, die Sucrosen und ihre Derivate, das Guarkernmehl, die Polyethylenglykole und ihre Gemische umfasst.

5. Sphäroid nach einem der Ansprüche 1 bis 4, **dadurch gekennzeichnet, dass** der Kern optional einen Verdünner, einen antistatischen Wirkstoff und/oder ein Gleitmittel umfasst.

6. Sphäroid nach einem der Ansprüche 1 bis 5, **dadurch gekennzeichnet, dass** das enterische Polymer aus der Gruppe ausgewählt ist, die das Celluloseacetatphthalat, das Hydroxypropylmethylcellulosephthalat, das Hydroxypropylmethylcellulosesukzinatphthalat, das Polyvinylacetatphthalat, das Celluloseacetattrimellitat, die Carboxymethylcellulose, den Shellack, allein oder im Gemisch verwendet, umfasst.

7. Sphäroid nach Anspruch 6, **dadurch gekennzeichnet, dass** das enterische Polymer ein Copolymer der Methacrylsäure ist.

8. Sphäroid nach einem der Ansprüche 1 bis 7, **dadurch gekennzeichnet, dass** die Fettsäuren des Gemischs gesättigter und/oder ungesättigter polyglycosylierter Glyceride von 8 bis 18 Kohlenstoffatome (C8-C18) umfassen.

9. Sphäroid nach Anspruch 8, **dadurch gekennzeichnet, dass** das Gemisch ein Gemisch aus Mono-, Di- und Triglyceriden und Polyethylenglycolmono- und -diester mit einem Molekulargewicht zwischen 200 und 1500 inklusive ist, eventuell aus Glycerol und freiem PEG, vornehmlich Palminstearinsäure umfasst, wobei das Gemisch durch einen Fusionspunkt zwischen 46,0 °C und 51,0 °C inklusive und einem Wert der hydrophilen und lipophilen Balance (HLB) gleich 13 **gekennzeichnet** ist.

10. Sphäroid nach Anspruch 8, **dadurch gekennzeichnet, dass** das Gemisch Gélucire® ist, insbesondere Gélucire 50/13.

11. Sphäroid nach Anspruch 1 bis 10, **dadurch gekennzeichnet, dass** der elastische und deformierbare Film optional einen Weichmacher umfasst, der aus der Gruppe ausgewählt ist, die das Triethylcitrat, das Acetyltributylcitrat, das Triacetin, das Tributylcitrat, das Diethylphthalat, die Polyethylenglycole, die Polysorbate, die Mono- und -diacetylglyceride, vorzugsweise das Triethylcitrat, umfasst.

12. Sphäroid nach Anspruch 1 bis 11, **dadurch gekennzeichnet, dass** die umhüllende Zusammensetzung optional einen grenzflächenaktiven Stoff, ein antistatisches Mittel, ein Gleitmittel umfasst.

13. Sphäroid nach Anspruch 1 bis 12, **dadurch gekennzeichnet, dass** das Sprengmittel aus der Gruppe ausgewählt ist, die die retikulierte NatriumCarboxymethylcellulose, die in der Fachwelt mit dem Begriff Croscarmellose bezeichnet wird, das Crospovidon, die Natrium-Carboxymethylstärke und ihre Gemische umfasst.

14. Sphäroid nach Anspruch 1 bis 13, **dadurch gekennzeichnet, dass** die lösliche Außenschicht optional ein Bindemittel und einen Hilfsstoff umfasst, insbesondere das Mannitol.

15. Verfahren zur Herstellung von Sphäroiden nach den Ansprüchen 1 bis 14, **dadurch gekennzeichnet, dass** es die folgenden Schritte umfasst:
➢ Herstellung eines Kerns, der einen oder mehrere Wirkstoff/e und mindestens ein Bindemittel enthält,
➢ Umhüllung der derart hergestellten Kerne durch Zerstäuben der umhüllenden Zusammensetzung, die ein enterisches Polymer und ein Gemisch gesättigter und/oder ungesättigter polyglycosylierter Glyceride enthält, deren Fettsäuren mindestens 8 Kohlenstoffatome umfassen, vorzugsweise von 8 bis 18 Kohlenstoffatome (C8-C18),
➢ Umhüllung der gastroresistenten Sphäroide mit einer wasserlöslichen Außenschicht, die mindestens ein Sprengmittel enthält, und
➢ Trocknung der Sphäroide.

16. Herstellungsverfahren nach Anspruch 15, **dadurch gekennzeichnet, dass** der den Wirkstoff enthaltende Kern durch Granulierung, durch Montage auf neutral oder auch durch Extrusion-Sphäronisierung hergestellt wird.

17. Herstellungsverfahren nach Anspruch 15 und 16, **dadurch gekennzeichnet, dass** die Sphäroide im fluidisierten Luftbett hergestellt werden.

18. Multipartikulare Tablette, die die Sphäroide nach einem der Ansprüche 1 bis 17 enthält.

19. Multipartikulare Tablette nach Anspruch 18, **dadurch gekennzeichnet, dass** sie nicht mehr als zirka 5 Gew.-% insgesamt eines oder mehrerer Hilfsstoffs/e umfasst.

20. Multipartikulare Tablette nach einem der Ansprüche 18 und 19, **dadurch gekennzeichnet, dass** der Hilfsstoff ein Gleitmittel, ein antistatischer Stoff und/oder ein permeabilisierender Stoff ist.

21. Multipartikulare Tablette nach einem der Ansprüche 18 bis 20, **dadurch gekennzeichnet, dass** sie ein Sphäroidengemisch umfasst, das einen oder mehrere Wirkstoff/e und Placebo-Sphäroide enthält.

22. Verfahren zur Herstellung von multipartikularen Tabletten nach einem der Ansprüche 18 bis 19, **dadurch gekennzeichnet, dass** es die folgenden Schritte umfasst:
➢ Mischen der gastroresistenten Sphäroide mit nicht mehr als 5 Gew.-% insgesamt eines oder mehrerer Hilfsstoffs/e,
➢ Kompression des Gemischs, um eine Stückform zu erhalten.
